(19) Europäisches Patentamt
European Patent Office
Office européen des brevets

(11) Veröffentlichungsnummer: **0 011 142**
**B1**

(12) # EUROPÄISCHE PATENTSCHRIFT

(45) Veröffentlichungstag der Patentschrift: **29.12.82**

(51) Int. Cl.³: **C 07 D 471/04,** A 61 K 31/505 // (C07D471/04, 221/00, 239/00)

(21) Anmeldenummer: **79103974.6**

(22) Anmeldetag: **15.10.79**

(54) **Pyrido (2,1-b) chinazolinon-Derivate, Verfahren zu ihrer Herstellung und sie enthaltende pharmazeutische Präparate.**

(30) Priorität: **18.10.78 DE 2845766**

(43) Veröffentlichungstag der Anmeldung: **28.05.80 Patentblatt 80/11**

(45) Bekanntmachung des Hinweises auf die Patenterteilung: **29.12.82 Patentblatt 82/52**

(84) Benannte Vertragsstaaten: **AT BE CH DE FR GB IT LU NL SE**

(56) Entgegenhaltungen:
**DE-A-2 557 425**
**DE-A-2 742 708**
**US-A-4 033 961**
**US-A-4 066 767**
**US-A-4 104 389**

**Die Akte enthält technische Angaben, die nach dem Eingang der Anmeldung eingereicht wurden und die nicht in dieser Patentschrift enthalten sind.**

(73) Patentinhaber: **SCHERING AKTIENGESELLSCHAFT**
***Berlin und Bergkamen,***
**Müllerstrasse 170/178 Postfach 65 03 11,**
**D-1000 Berlin 65 (DE)**

(72) Erfinder: **Biere, Helmut, Dr., Zeltinger Strasse 15, D-1000 Berlin 28 (DE)**
Erfinder: **Kapp, Joachim-Friedrich, Dr., Ludolfinger Weg 51, D-1000 Berlin 28 (DE)**
Erfinder: **Böttcher, Irmgard, Dr., Rodelbahnpfad 5, D-1000 Berlin 28 (DE)**

**EP 0 011 142 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

BUNDESDRUCKEREI BERLIN

# Pyrido[2,1-b]chinazolinon-Derivate, Verfahren zu ihrer Herstellung und sie enthaltende pharmazeutische Präparate

Die Erfindung betrifft Pyrido[2,1-b]chinazolinon-Derivate der allgemeinen Formel I gemäß Anspruch 1, Verfahren zu ihrer Herstellung und ihre Verwendung als Arzneimittelwirkstoffe.

Die Erfindung betrifft weiters sowohl die racemischen Pyrido[2,1-b]chinazolinon-Derivate der allgemeinen Formel I, als auch deren optisch aktive Antipoden.

Als physiologisch verträgliche Salze der Carboxylgruppe X in der allgemeinen Formel I seien beispielsweise die Alkali- oder Erdalkalimetallsalze, wie das Natriumsalz oder das Calciumsalz, das Ammoniumsalz, das Kupfer(II)-salz oder das Methylglucaminsalz, sowie die Salze dieser Verbindungen mit Aminosäuren genannt.

Die Alkoxycarbonylgruppe X kann als Alkoxyrest beispielsweise den Methyl-, Äthyl-, Propyl-, Isopropyl-, Butyl-, sek.-Butyl-, tert.-Butyl-, Pentyl-, Isopentyl-, tert.-Pentyl- oder Hexylrest enthalten.

Salze der Pyrido[2,1-c]chinazolinon-Derivate der allgemeinen Formel I mit physiologisch unbedenklichen Basen sind beispielsweise Alkalimetallsalze oder Erdalkalimetallsalze, wie die Natriumsalze oder Calciumsalze, oder Amoniumsalze, Kupfer(II)-salze, Methylglucaminsalze, Piperazinsalze, oder die Salze dieser Verbindungen mit Aminosäuren.

Als Pyrido[2,1-b]chinazolinium Salze der Verbindungen der allgemeinen Formel I kommen beispielsweise die Hydrochloride, Hydrobromide, Sulfate, Phosphate, Maleate, Tartrate oder Citrate in Betracht.

Die Pyrido[2,1-b]chinazolinon-Derivate der allgemeinen Formel I sind pharmakologisch wirksame Substanzen oder Zwischenprodukte zu ihrer Herstellung. Die pharmakologisch wirksamen Verbindungen zeichnen sich insbesondere durch eine antiphlogistische, antipyretische, analgetische und antiallergische Wirksamkeit aus. Darüber hinaus bewirken diese Verbindungen in vitro eine ausgeprägte Phophodiesterasehemmung. Die Magenverträglichkeit der wirksamen Pyrido[2,1-b]chinazolinon-Derivate ist relativ gut, ihre Toxizität relativ gering.

Im Vergleich zu den aus der deutschen Patentanmeldung 2 557 427 und den US-Patentschriften 4 033 961 und 4 104 389 vorbekannten strukturanalogen Verbindungen zeichnen sich die erfindungsgemäßen Substanzen durch eine überlegene antiinflammatorische Wirksamkeit aus, wie man mit Hilfe des Carragenin-Pfotenödem-Test nachweisen kann.

Die pharmakologisch wirksamen Verbindungen eignen sich in Kombination mit den in der galenischen Pharmazie üblichen Trägermitteln zum Beispiel zur Behandlung von akuten und chronischen Entzündungsprozessen, Polyarthritis, Neurodermitis, Asthma bronchiale, Heufieber, Dysmenorrhö und Migräne.

Die Herstellung der Arzneimittelspezialitäten erfolgt in üblicher Weise, indem man die Wirkstoffe mit geeigneten Zusätzen, Trägersubstanzen und Geschmackskorrigentien in die gewünschten Applikationsformen, wie Tabletten, Dragees, Kapseln, Lösungen und Inhalationsmitteln überführt.

Für die orale Anwendung eignen sich insbesondere Tabletten, Dragees und Kapseln, welche beispielsweise 5 bis 500 mg Wirkstoff und 50 mg bis 2 g pharmakologisch unwirksamen Trägers, wie zum Beispiel Laktose, Amylose, Talkum, Gelatine, Magnesiumstearat sowie die üblichen Zusätze enthalten.

Das erfindungsgemäße Verfahren zur Herstellung der Pyrido[2,1-b]chinazolinon-Derivate gemäß Anspruch 31 wird unter Bedingungen durchgeführt, die dem Fachmann wohlbekannt sind (Ullmann-Reaktion). Als Ausgangsverbindungen für dieses Verfahren werden vorzugsweise solche Pyridinderivate der allgemeinen Formel II verwendet, die als Substituenten Y eine Aminogruppe, ein Chloratom, ein Bromatom, eine Alkoxygruppe mit 1 bis 4 Kohlenstoffatomen im Alkylrest, eine Trimethylsilyloxygruppe, eine Methansulfonyloxygruppe oder eine p-Toluolsulfonyloxygruppe besitzen. Die als Ausgangsverbindungen verwendeten Phenylderivate der allgemeinen Formel III haben vorzugsweise als Substituent V ein Wasserstoffatom oder eine Alkylgruppe mit 1 bis 4 Kohlenstoffatomen und als Substituent Z eine Aminogruppe, ein Chloratom oder ein Bromatom. Unter Alkoxycarbonylgruppen $X_1$ der Verbindungen III sind vorzugsweise Gruppen mit 1 bis 4 Kohlenstoffatomen im Alkylrest zu verstehen.

Die Reaktion wird vorzugsweise durchgeführt, indem man die Reaktionspartner — gewünschtenfalls in einem hochsiedenden Lösungsmittel (wie zum Beispiel Äthylenglykol, Äthylenglykolmonomethyläther, Äthylenglykolmonobutyläther oder Äthylenglykoldiäthyläther) in Gegenwart von Basen mit Kupfer oder Zinkkatalysatoren auf 100 bis 250°C erhitzt. Geeignete Basen sind beispielsweise Alkalikarbonate (Natriumkarbonat oder Kaliumkarbonat) oder hochsiedende tertiäre Amine (N-Äthylmorpholin, N-Äthylpiperidin). Als geeignete Kupfer- oder Zinkkatalysatoren seien beispielsweise Zink(II)-chlorid, Kupferpulver, Kupfer(I)-oxid, Kupfer(II)-oxid, Kupfer(II)-chlorid, Kupfer(II)-sulfat oder insbesondere Kupfer(II)-bromid und Kupfer(I)-bromid genannt.

Das erfindungsgemäße Verfahren gemäß Patentanspruch 32a) erfolgt ebenfalls unter den Bedingungen, wie sie dem Fachmann wohlbekannt sind. So kann man die Nitrile beispielsweise mit starken Mineralsäuren (wie Salzsäure oder Schwefelsäure) oder mit starken Basen (wie wäßrige Natronlauge oder Kalilauge) partiell zu den entsprechenden Amiden oder unter verschärften Bedingungen zu den entsprechenden Carbonsäuren hydrolysieren.

Für diese Reaktion kann die wasserhaltige Mineralsäure oder Base selbst als Lösungsmittel verwendet werden. Es ist aber andererseits auch möglich, die Reaktion in Gegenwart von polaren Lösungsmitteln wie zum Beispiel niederen Alkoholen (Methanol, Äthanol, Isopropanol), Carbonsäuren (Essigsäure, Propionsäure), polaren Äthern (Glykolmonomethyläther, Dioxan, Tetrahydrofuran) oder dipolaren aprotischen Lösungsmitteln (Dimethylsulfoxyd) durchzuführen.

Üblicherweise wird die Hydrolyse bei einer Reaktionstemperatur von 20 bis 160° C durchgeführt.

Die für diese Reaktion verwendeten Ausgangsverbindungen der allgemeinen Formel Ia können in an sich bekannter Weise hergestellt werden.

Das erfindungsgemäße Verfahren gemäß Patentanspruch 32b) wird ebenfalls unter den Bedingungen durchgeführt, die dem Fachmann wohlbekannt sind. Diese Reaktion wird durch thermische Erhitzung der Malonsäure-Derivate der allgemeinen Formel IV auf 50 bis 150°C durchgeführt, wobei man die Decarboxylierung in Abwesenheit eines Lösungsmittels oder auch in Anwesenheit eines hochsiedenden Lösungsmittels (wie Xylol, Chlorbenzol oder Dekalin) durchführen kann.

Die für diese Verfahrensvariante verwendeten Ausgangsverbindungen der allgemeinen Formel IV können beispielsweise wie folgt hergestellt werden: Pyrido[2,1-b]chinazolinon-Derivate der allgemeinen Formel I mit $R_1$ in der Bedeutung von Wasserstoff und X in der Bedeutung einer Cyanogruppe oder Alkoxycarbonylgruppe werden in Diäthylcarbonat mit Natriumhydrid umgesetzt. Man destilliert das überschüssige Lösungsmittel ab und kann dann gegebenenfalls das so erhaltene Rohprodukt in Dimethylformamid mit Methyljodid und Natrium methylieren.

Das erfindungsgemäße Verfahren gemäß Patentanspruch 32c) erfolgt ebenfalls unter den Bedingungen, die dem Fachmann wohlbekannt sind. So kann man beispielsweise die Verbindungen der allgemeinen Formel V in einem inerten Lösungsmittel (Dioxan, Tetrahydrofuran, Glykoldimethyläther) mit Lithium-diisopropylamid und Kohlendioxid umsetzen. Andererseits ist es beispielsweise auch möglich die Verbindungen der allgemeinen Formel I in einem inerten Lösungsmittel (Dioxan, Tetrahydrofuran, Glykoldimethyläther) mit Kaliumhydrid und Chlorkohlensäureäthylester umzusetzen.

Die für diese Verfahrensvariante benötigten Ausgangsverbindungen der allgemeinen Formel V können beispielsweise nach dem Verfahren gemäß Patentanspruch 31 hergestellt werden.

Die übrigen Gegebenenfallsmaßnahmen gemäß Patentanspruch 31 oder 32 können beispielsweise unter den Bedingungen durchgeführt werden, die in der DE-OS 2 431 292 beschrieben sind.

Die nachfolgenden Beispiele dienen zur Erläuterung des erfindungsgemäßen Verfahrens.

## Beispiel 1

$a_1$) Eine Lösung von 15,1 g 2-Amino-5-methylbenzoesäure in 200 ml Ethylenglykolmonobutylether wird mit 7,6 g gepulvertem Kaliumkarbonat, 15 g N-Ethylmorpholin, 28 g 2,5-Dibrompyridin und 1,8 g Kupfer(II)-bromid versetzt und unter Argon 6 Stunden bei 180°C gerührt. Nach Abdestillieren des Lösungsmittels im Wasserstrahlvakuum wird der Rückstand mit 1 l Ethylacetat aufgenommen und zuerst mit 1 N-Essigsäure, dann mit Natriumhydrogencarbonatlösung gewaschen. Die organische Phase wird eingeengt und der Rückstand aus Chloroform umkristallisiert. Man erhält 15,2 g 8-Brom-2-methyl-11H-pyrido[2,1-b]chinazolin-11-on vom Schmelzpunkt 204°C.

$a_2$) Eine Lösung von 1,7 g 2-Chlor-5-methylbenzoesäure in 30 ml Diethylenglykoldimethylether wird mit 1,5 g N-Ethylmorpholin, 0,7 g gepulvertem Kaliumkarbonat, 100 mg Kupfer(II)-bromid und 2 g 2-Amino-5-brom-pyridin versetzt und die Mischung unter Stickstoff 14 Stunden auf 160°C erhitzt. Nach Abdestillieren des Lösungsmittels im Vakuum wird analog $a_1$) aufgearbeitet. Man erhält 1,2 g 8-Brom-2-methyl-11H-pyrido[2,1-b]chinazolin-11-on.

b) Eine Lösung von 14,8 g 8-Brom-2-methyl-11H-pyrido[2,1-b]chinazolin-11-on in 500 ml Tetrachlorkohlenstoff wird mit 11 g Bromsuccinimid und 0,5 g Azobisisobutyronitril versetzt und mit einer 500 Watt Lampe bestrahlt, wobei die Lösung unter Rückfluß kocht. Nach dem Einengen der Lösung wird der Rückstand in ca. 2 l Chloroform aufgenommen, die organische Phase wird mit Wasser gewaschen, und nach dem Abdestillieren des Lösungsmittels erhält man 18 g 8-Brom-2-brommethyl-11H-pyrido[2,1-b]chinazolin-11-on vom Schmelzpunkt 208°C (Toluol).

$c_1$) 3,7 g 8-Brom-2-brommethyl-11H-pyrido[2,1-b]chinazolin-11-on werden zu einer Lösung von 1,5 g Natriumcyanid, 0,2 g Kaliumjodid, 3 ml Wasser und 50 ml Ethanol gegeben und die Mischung 15 Minuten unter Rückfluß gekocht. Nach dem Abkühlen kristallisiert das gebildete Nitril aus; es wird abgesaugt, mit Wasser und wenig Ethanol gewaschen und aus Acetonitril umkristallisiert. Man erhält 2,2 g 8-Brom-11-oxo-11H-pyrido[2,1-b]chinazolin-2-acetonitril vom Schmelzpunkt 240°C.

$c_2$) Eine Lösung von 17,3 g 8-Brom-2-brommethyl-11H-pyrido[2,1-b]chinazolin-11-on in 500 ml Chloroform wird mit dem vorher bereiteten Komplex aus 3,1 g Kaliumcyanid und 16,9 g Dibenzo-18-krone-6 sowie weiteren 3,1 g Kaliumcyanid versetzt und 30 Minuten unter Rückfluß

gekocht, anschließend über Nacht bei Raumtemperatur gerührt. Die Lösung wird dann über eine Kieselgelsäule mit Toluol chromatographiert. Man erhält 8,5 g 8-Brom-11-oxo-11H-pyrido[2,1-b]-chinazolin-2-acetonitril mit Schmelzpunkt 240° C (Tetrahydrofuran-Wasser).

d) Eine Lösung von 41,7 g konzentrierter Schwefelsäure und 23 ml Wasser wird mit 4 g 8-Brom-11-oxo-11H-pyrido[2,1-b]chinazolin-2-acetonitril versetzt und diese 3 Stunden bei 120°C gerührt. Nach dem Abkühlen wird die Lösung in ein Gemisch von 400 ml Eiswasser gegossen und mit Natriumacetat auf pH 4 bis 5 eingestellt. Man erhält ein Kristallisat von 3,9 g 8-Brom-11-oxo-11H-pyrido[2,1-b]chinazolin-2-essigsäure mit einem Schmelzpunkt von 271°C (Dimethylformamid-Wasser).

Beispiel 2

a) Eine Lösung von 3,8 g 8-Brom-11-oxo-11H-pyrido[2,1-b]chinazolin-2-acetonitril in 75 ml Diethylcarbonat wird mit 1,2 g Natriumhydrid-dispersion (50%) versetzt und 2 Stunden unter Rückfluß gekocht. Nach dem Abdestillieren des Lösungsmittels wird der Rückstand in 50 ml Dimethylformamid aufgenommen und bei 0° C mit 3,5 g Methyljodid versetzt. Die Mischung wird 1 Stunde bei 0° C, dann über Nacht bei Raumtemperatur gerührt. Nach Einengen im Hochvakuum wird der ölige Rückstand mit 250 ml 1 N Essigsäure sowie 250 ml Wasser versetzt und das organische Material mit Ethylacetat extrahiert. Das Rohprodukt wird aus Ethanol umkristallisiert und man erhält 2,5 g 2-Cyano-2-(8-brom-11-oxo-11H-pyrido[2,1-b]chinazolin-2-yl)-propionsäure-ethylester mit Schmelzpunkt 156° C.

b) Eine Mischung von 1 g 2-Cyano-2-(8-brom-11-oxo-11H-pyrido[2,1-b]chinazolin-2-yl)-propionsäure-ethylester, 4 ml Wasser und 3 ml Schwefelsäure (konz.) wird 3 Stunden bei 120°C (Badtemperatur) gerührt. Nach Abkühlen wird die Mischung mit Eiswasser verdünnt und mit Chloroform extrahiert. Der Rückstand wird aus Ethanol umkristallisiert. Man erhält 0,5 g 2-(8-Brom-11-oxo-11H-pyrido[2,1-b]chinazolin-2-yl)-propionsäure mit Schmelzpunkt 246° C.

Beispiel 3

a) Eine Lösung von 2,1 g 2-(4-Amino-3-carboxy-phenyl)-propionsäure in 5 ml Ethylenglykolmonobutylether wird mit 1,4 g gepulvertem Kaliumkarbonat und 1,2 g N-Ethylmorpholin 0,5 Stunden bei Raumtemperatur gerührt. Dann werden 3,0 g 2,5-Dibrompyridin sowie 150 mg Kupfer(II)-bromid zugesetzt und die Mischung unter Stickstoff 7 Stunden auf 180° C (Badtemperatur) erhitzt. Nach dem Abdestillieren des Lösungsmittels im Vakuum wird der Rückstand in Essigester gelöst und mit verdünnter Essigsäure gewaschen. Nach Säure-Base-Extraktion wird die organische Säurefraktion aus Ethanol umkristallisiert und man erhält 1,5 g 2-(8-Brom-11-oxo-11H-pyrido[2,1-b]chinazolin-2-yl)-propionsäure mit Schmelzpunkt 251° C (Dimethylformamid-Wasser).

b) Die Herstellung des Ausgangsmaterials 2-(4-Amino-3-carboxyphenyl)-propionsäure bzw. seines Esters erfolgt auf folgende Weise:

$b_1$) Zu einer Lösung aus 35,7 g Chloralhydrat in 480 ml Wasser werden 36,9 g Natriumsulfat, 38,6 g 2-(4-Aminophenyl)-propionsäure-ethylester [hergestellt nach Lit.: G. Nannini et al., Arzneimittelforschung 23, 1090 (1973)], 120 ml Wasser, 17 ml konzentrierte Salzsäure und eine Lösung aus 43,9 g Hydroxylammoniumchlorid in 200 ml Wasser zugefügt und das Reaktionsgemisch langsam über 45 Minuten zum Sieden erhitzt, dann 10 Minuten am Siedepunkt gehalten und abgekühlt. Das sich ölig abscheidende Produkt wird mit Ethylacetat aufgenommen und mit Wasser gewaschen. Nach Säure-Base-Trennung wird die organische Säurefraktion eingeengt und aus Acetonitril umkristallisiert. Man erhält 25 g 2-[4-(2-Hydroxyimino)-acetamido-phenyl]-propionsäure mit Schmelzpunkt 163° C.

$b_2$) 13 g 2-[4-(2-Hydroxyimino)-acetamido-phenyl]-propionsäure werden portionsweise in 55 g Schwefelsäure (Dichte 1,84) eingetragen, die auf 50°C vorgewärmt wurde. Dabei wird eine Temperatur von 70°C erreicht. Nach beendeter Zugabe wird noch 10 Minuten auf 80°C erhitzt, dann in 300 ml Eiswasser eingegossen und mit Ethylacetat extrahiert. Nach Umkristallisation aus Acetonitril werden 10 g 2-(5-Isatinyl)-propionsäure mit Schmelzpunkt 224° C erhalten.

$b_3$) Zu einer Lösung aus 2,2 g 2-(5-Isatinyl)-propionsäure in 22 ml Wasser und 3 ml 32%iger Natronlauge wird tropfenweise während einer Zeitdauer von 20 Minuten 2,9 g Wasserstoffperoxid (30%) zugefügt und anschließend noch 20 Minuten gerührt. Nach Ansäuern des Reaktionsgemisches mit 2 N Salzsäure (pH 2—3) wird das organische Material mit Ethylacetat extrahiert und

schließlich aus Acetonitril umkristallisiert. Man erhält 1,8 g 2-(4-Amino-3-carboxy-phenyl)-propionsäure mit Schmelzpunkt 179° C.

$b_4$) 3 g 2-(4-Amino-3-carboxy-phenyl)-propionsäure werden in 500 ml methanolischer Salzsäure (ca. 3%) gelöst und 2 Tage bei Raumtemperatur stehengelassen. Nach Einengen wird der Rückstand in Ethylacetat aufgenommen, mit Natriumhydrogenkarbonatlösung gewaschen und umkristallisiert. Man erhält 2,8 g 2-(4-Amino-3-carboxy-phenyl)-propionsäure-methylester mit Schmelzpunkt 151° C (Acetonitril).

Beispiel 4

a) Unter den Bedingungen des Beispiels 3a) wird aus 2-Brom-5-chlorpyridin und 2-(4-Amino-3-carboxyphenyl)-propionsäure 2-(8-Chlor-11-oxo-11H-pyrido[2,1-b]chinazolin-2-yl)-propionsäure mit Schmelzpunkt 234° C erhalten.

b) Das Ausgangsmaterial 2-Brom-5-chlorpyridin wird analog literaturbekanntem Verfahren auf folgendem Weg dargestellt:
Zu einer Lösung von 14 g 2-Amino-5-chlorpyridin in 30 ml Wasser und 45 ml Bromwasserstoffsäure (63% in Wasser) werden 17 ml Brom hinzugefügt und das Gemisch auf 0° C gekühlt. Unter Temperaturkontrolle wird eine Lösung von 21 g Natriumnitrit in 30 ml Wasser so schnell zugetropft, daß die Temperatur der Mischung 5° C nicht überschreitet. Anschließend wird unter Kühlen mit einer Lösung aus 45 g Natriumhydroxid in 115 ml Wasser versetzt. Die ausfallenden Kristalle werden abgesaugt, mit Wasser gewaschen und zunächst aus Aceton, dann noch einmal aus Ethanol umkristallisiert. Man erhält 10 g 2-Brom-5-chlorpyridin mit Schmelzpunkt 68° C.

Beispiel 5

a) Unter den Bedingungen des Beispiels 3a) wird aus 2,3-Dichlorpyridin und 2-(4-Amino-3-carboxyphenyl)-propionsäure-methylester 2-(6-Chlor-11-oxo-11H-pyrido[2,1-b]chinazolin-2-yl)-propionsäure-methylester erhalten.

b) Dieser wird durch Erhitzen mit verdünnter Natronlauge in die 2-(6-Chlor-11-oxo-11H-pyrido[2,1-b]chinazolin-2-yl)-propionsäure überführt.

Beispiel 6

Unter den Bedingungen des Beispiels 3a) wird aus 2-Brom-5-fluorpyridin (hergestellt nach R. A. Abramovitch et al., J. Org. Chem. 39, 1802 [1974]) und 2-(4-Amino-3-carboxyphenyl)-propionsäure 2-(8-Fluor-11-oxo-11H-pyrido[2,1-b]chinazolin-2-yl)-propionsäure erhalten. Schmelzpunkt 221° C (Dimethylformamid-Wasser).

Beispiel 7

Unter den Bedingungen des Beispiels 3a) wird aus 2-Brom-3,5-dichlorpyridin (hergestellt aus 2-Amino-3,5-dichlorpyridin nach Beispiel 4b) und 2-(4-Amino-3-carboxy-phenyl)-propionsäure-methylester der 2-(6,8-Dichlor-11-oxo-11H-pyrido[2,1-b]chinazolin-2-yl)-propionsäure-methylester erhalten. Schmelzpunkt 138° C (Äther-Acetonitril).

Beispiel 8

Eine Mischung von 1,4 g 2-(6,8-Dichlor-11-oxo-11H-pyrido[2,1-b]chinazolin-2-yl)-propionsäure-methylester, 10 ml Wasser und 10 ml konzentrierter Schwefelsäure wird 4 Stunden bei 120° C (Badtemperatur) gerührt. Nach dem Abkühlen wird mit 10 ml Eiswasser verdünnt und mit Natronlauge auf einen pH von 4—5 eingestellt. Nach Extraktion mit Ethylacetat, Trocknen und Einengen der organischen Phase werden 1,1 g 2-(6,8-Dichlor-11-oxo-11H-pyrido[2,1-b]chinazolin-2-yl)-propionsäure erhalten. Schmelzpunkt 241° C (Dimethylformamid-Wasser).

Beispiel 9

a) Unter den Bedingungen des Beispiels 3a) wird aus 2-Chlor-4-methylpyridin und 2-(4-Amino-3-carboxy-phenyl)-propionsäure-methylester der 2-(7-Methyl-11-oxo-11H-pyrido[2,1-b]chinazolin-2-yl)-propionsäure-methylester erhalten. Schmelzpunkt 120° C (Acetonitril).

b) Dieser wird unter den Bedingungen des Beispiels 8 zur 2-(7-Methyl-11-oxo-11H-pyrido[2,1-b]chinazolin-2-yl)-propionsäure hydrolysiert. Schmelzpunkt 267° C (Essigsäure).

Beispiel 10

a) Unter den Bedingungen des Beispiels 3a wird aus 2-Chlor-6-methylpyridin und 2-(4-Amino-3-carboxy-phenyl)-propionsäure-methylester der 2-(9-Methyl-11-oxo-11H-pyrido[2,1-b]chinazolin-2-yl)-propionsäure-methylester erhalten.

b) Dieser wird unter den Bedingungen des Beispiels 8 zur 2-(9-Methyl-11-oxo-11H-pyrido[2,1-b]chinazolin-2-yl)-propionsäure hydrolysiert.

Beispiel 11

a) Unter den Bedingungen des Beispiels 3a wird aus 2-Brom-5-methylpyridin und 2-(4-Amino-3-carboxyphenyl)-propionsäure-methylester der 2-(8-Methyl-11-oxo-11H-pyrido[2,1-b]chinazolin-2-yl)-propionsäure-methylester erhalten. Schmelzpunkt 151° C (Acetonitril).

b) Dieser wird unter den Bedingungen des Beispiels 8 zur 2-(8-Methyl-11-oxo-11H-pyrido[2,1-b]chinazolin-2-yl)-propionsäure hydrolysiert. Schmelzpunkt 233° C (Acetonitril).

Beispiel 12

Unter den Bedingungen des Beispiels 3a werden 5,2 g 2-(4-Amino-3-carboxy-phenyl)-propionsäure-methylester mit 4,7 g 2-Brom-5-methylthio-pyridin umgesetzt, aufbereitet und man erhält 3,3 g 2-(8-Methylthio-11-oxo-11H-pyrido[2,1-b]chinazolin-2-yl)-propionsäuremethylester vom Schmelzpunkt 113 – 114° C (Diäthyläther).

Beispiel 13

1,3 g 2-(8-Methylthio-11-oxo-11H-pyrido[2,1-b]chinazolin-2-yl)-propionsäuremethylester werden wie in Beispiel 8 beschrieben verseift, aufbereitet und man erhält 1,0 g 2-(8-Methylthio-11-oxo-11H-pyrido[2,1-b]chinazolin-2-yl)-propionsäure vom Schmelzpunkt 213—214° C (Dimethylformamid-Wasser).

Beispiel 14

190 mg 2-(8-Methylthio-11-oxo-11H-pyrido[2,1-b]chinazolin-2-yl)-propionsäure werden mit 10 ml Eisessig und 5 ml Chloroform versetzt. Man gibt zu der Mischung 70 mg 30%iges Wasserstoffsuperoxid, rührt zwei Tage lang bei Raumtemperatur, setzt nochmals 7 mg 30%iges Wasserstoffsuperoxid zu und rührt nochmals einen Tag lang. Die Reaktionsmischung wird im Vakuum eingeengt, der Rückstand aus Methanol umkristallisiert und man erhält 140 mg 2-(8-Methylsulfinyl-11-oxo-11H-pyrido[2,1-b]chinazolin-2-yl)-propionsäure vom Schmelzpunkt 225 – 226° C.

Beispiel 15

Unter den Bedingungen des Beispiels 18 werden 2,85 g 2-(8-Methylthio-11-oxo-11H-pyrido[2,1-b]chinazolin-2-yl)-propionsäuremethylester oxydiert, aufbereitet und man erhält 2,2 g 2-(8-Methylsulfinyl-11-oxo-11H-pyrido[2,1-b]chinazolin-2-yl)-propionsäuremethylester vom Schmelzpunkt 160 – 162° C (Methanol).

Beispiel 16

1,1 g 2-(8-Methylsulfinyl)-11-oxo-11H-pyrido[2,1-b]chinazolin-2-yl)-propionsäuremethylester werden mit 20 ml Polyphosphorsäure versetzt, auf 80° C erwärmt, portionsweise mit 335 mg Natriumazid versetzt, 8 Stunden lang bei 80° C und 16 Stunden lang bei Raumtemperatur gerührt.

Dann verdünnt man die Reaktionsmischung mit Wasser, neutralisiert sie mit konzentriertem Ammoniak, sättigt sie mit Kochsalz und extrahiert sie mit Ethylacetat. Die organische Phase wird eingeengt, der Rückstand aus Methanol-Diisopropylether umkristallisiert und man erhält 660 mg

2-(8-Methylsulfonimidoyl-11-oxo-11H-pyrido[2,1-b]chinazolin-2-yl)-propionsäuremethylester vom Schmelzpunkt 158 – 160° C.

Beispiel 17

Unter den Bedingungen des Beispiels 3 wird aus 2,5-Dibrompyridin und 2-(4-Amino-3-carboxyphenyl)-propionsäuremethylester der 2-(8-Brom-11-oxo-11H-pyrido-[2,1-b]chinazolin-2-yl)-propionsäuremethylester vom Schmelzpunkt 172° C (Methanol) erhalten.

Beispiel 18

Unter den Bedingungen des Beispiels 3 wird aus 2-Brom-5-chlorpyridin und 2-(4-Amino-3-carboxyphenyl)-propionsäuremethylester der 2-(8-Chlor-11-oxo-11H-pyrido[2,1-b]chinazolin-2-yl)-propionsäuremethylester vom Schmelzpunkt 173° C (Ether) erhalten.

Beispiel 19

Unter den Bedingungen des Beispiels 3 wird aus 2-Brom-5-fluorpyridin und 2-(4-Amino-3-carboxyphenyl)-propionsäuremethylester der 2-(8-Fluor-11-oxo-11H-pyrido[2,1-b]chinazolin-2-yl)-propionsäuremethylester vom Schmelzpunkt 138° C (Ether) erhalten.

Beispiel 20

100 mg 2-(8-Methylthio-11-oxo-11H-pyrido[2,1-b]chinazolin-2-yl)-propionsäure werden unter den Bedingungen des Beispiels 18 mit 250 mg 30%igem Wasserstoffperoxid umgesetzt, aufbereitet und man erhält 45 mg 2-(8-Methylsulfonyl-11-oxo-11H-pyrido[2,1-b]chinazolin-2-yl)-propionsäure vom Schmelzpunkt 243° C.

**Patentansprüche**

1. Pyrido[2,1-b]chinazolinon-Derivate der allgemeinen Formel I

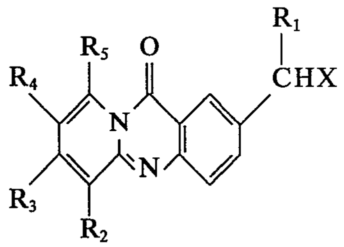

(I)

worin

X eine Cyanogruppe, eine Carbamoylgruppe, eine Alkoxycarbonylgruppe mit 1 bis 6 Kohlenstoffatomen im Alkylrest oder eine Carboxylgruppe und deren Salze mit physiologisch verträglichen Basen,

$R_1$ ein Wasserstoffatom oder eine Methylgruppe und

$R_2$ bis $R_5$ Wasserstoffatome, Halogenatome, 1 bis 4 Kohlenstoffatome enthaltende Alkylgruppen, Methylthiogruppen, Methylsulfinylgruppen oder Methylsulfonylgruppen, mit der Maßgabe, daß 2 oder 3 der Gruppen $R_2$ bis $R_5$ Wasserstoffatome darstellen, sowie deren Salze mit physiologisch unbedenklichen Säuren oder Basen.

2. Pyrido[2,1-b]chinazolinon-Derivate der allgemeinen Formel I gemäß Anspruch 1, worin 1 oder 2 der Gruppen $R_2$ bis $R_5$ Fluoratome, Chloratome, Bromatome und/oder Methylgruppen bedeuten.

3. Pyrido[2,1-b]chinazolinon-Derivate der allgemeinen Formel I gemäß Anspruch 1 und 2, worin X eine Carboxylgruppe, oder deren Alkalimetallsalz, Erdalkalimetallsalz, Kupfer(II)-salz oder Methylglucaminsalz bedeutet.

4. 2-(8-Brom-11-oxo-11H-pyrido[2,1-b]chinazolin-2-yl)-propionsäuremethylester.

5. 2-(8-Chlor-11-oxo-11H-pyrido[2,1-b]chinazolin-2-yl)-propionsäuremethylester.

6. 2-(8-Fluor-11-oxo-11H-pyrido[2,1-b]chinazolin-2-yl)-propionsäuremethylester.

7. 2-(8-Mesylamino-11-oxo-11H-pyrido[2,1-b]chinazolin-2-yl)-propionsäuremethylester.

8. 8-Brom-11-oxo-11H-pyrido[2,1-b]chinazolin-2-acetonitril.
9. 8-Brom-11-oxo-11H-pyrido[2,1-b]chinazolin-2-essigsäure.
10. 2-(8-Brom-11-oxo-11H-pyrido[2,1-b]chinazolin-2-yl)-propionsäure.
11. 2-(8-Chlor-11-oxo-11H-pyrido[2,1-b]chinazolin-2-yl)-propionsäure.
12. 2-(6-Chlor-11-oxo-11H-pyrido[2,1-b]chinazolin-2-yl)-propionsäure-methylester.
13. 2-(6-Chlor-11-oxo-11H-pyrido[2,1-b]chinazolin-2-yl)-propionsäure.
14. 2-(8-Fluor-11-oxo-11H-pyrido[2,1-b]chinazolin-2-yl)-propionsäure.
15. 2-(6,8-Dichlor-11-oxo-11H-pyrido[2,1-b]chinazolin-2-yl)-propionsäure-methylester.
16. 2-(6,8-Dichlor-11-oxo-11H-pyrido[2,1-b]chinazolin-2-yl)-propionsäure.
17. 2-(7-Methyl-11-oxo-11H-pyrido[2,1-b]chinazolin-2-yl)-propionsäure-methylester.
18. 2-(7-Methyl-11-oxo-11H-pyrido[2,1-b]chinazolin-2-yl)-propionsäure.
19. 2-(9-Methyl-11-oxo-11H-pyrido[2,1-b]chinazolin-2-yl)-propionsäure-methylester.
20. 2-(9-Methyl-11-oxo-11H-pyrido[2,1-b]chinazolin-2-yl)-propionsäure.
21. 2-(8-Methyl-11-oxo-11H-pyrido[2,1-b]chinazolin-2-yl)-propionsäure-methylester.
22. 2-(8-Methyl-11-oxo-11H-pyrido[2,1-b]chinazolin-2-yl)-propionsäure.
23. 2-(8-Brom-7-methyl-11-oxo-11H-pyrido[2,1-b]chinazolin-2-yl)-propionsäure-methylester.
24. 2-(8-Brom-7-methyl-11-oxo-11H-pyrido[2,1-b]chinazolin-2-yl)-propionsäure.
25. 2-(8-Chlor-6-methyl-11-oxo-11H-pyrido[2,1-b]chinazolin-2-yl)-propionsäure.
26. 2-(8-Methylthio-11-oxo-11H-pyrido[2,1-b]chinazolin-2-yl)-propionsäuremethylester.
27. 2-(8-Methylthio-11-oxo-11H-pyrido-[2,1-b]chinazolin-2-yl)-propionsäure.
28. 2-(8-Methylsulfinyl-11-oxo-11H-pyrido[2,1-b]chinazolin-2-yl)-propionsäure.
29. 2-(8-Methylsulfinyl-11-oxo-11H-pyrido[2,1-b]chinazolin-2-yl)-propionsäuremethylester.

30. Pharmazeutische Präparate, gekennzeichnet durch einen Gehalt von ein oder zwei Pyrido[2,1-b]chinazolinon-Derivaten gemäß Anspruch 1 bis 29.

31. Verfahren zur Herstellung von Pyrido[2,1-b]chinazolinon-Derivaten gemäß Anspruch 1, dadurch gekennzeichnet, daß man ein Pyridinderivat der allgemeinen Formel II mit einem Phenylderivat der allgemeinen Formel III

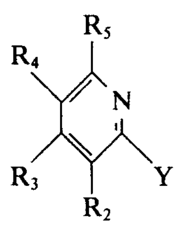

(II)

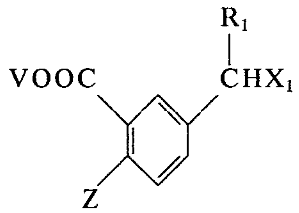

(III)

worin

$R_1$ bis $R_5$ die im Anspruch 1 genannte Bedeutung besitzen und
$X_1$ eine Cyanogruppe, eine Carboxylgruppe oder eine Alkoxycarbonylgruppe,
V ein Wasserstoffatom oder eine Alkylgruppe,
Y eine Aminogruppe und Z ein Halogenatom oder
Y ein Halogenatom, eine veresterte oder verätherte Hydroxygruppe und
Z eine Aminogruppe bedeuten,

kondensiert, gegegebenenfalls die so entstandene Cyanoverbindung oder Alkoxycarbonylverbindung verseift oder in die Carbamoylverbindung überführt, sowie gegebenenfalls die racemische Carbonsäure in ihre optischen Antipoden aufspaltet und/oder die Carbonsäuren oder reaktionsfähige Derivate derselben in ihre Salze, Ester, oder Amide überführt und gewünschtenfalls die so erhaltenen Pyrido[2,1-b]chinazolinon-Derivate mit physiologisch unbedenklichen Säuren und Basen umsetzt.

32. Verfahren zur Herstellung von Pyrido[2,1-b]chinazolinon-Derivaten der allgemeinen Formel I a

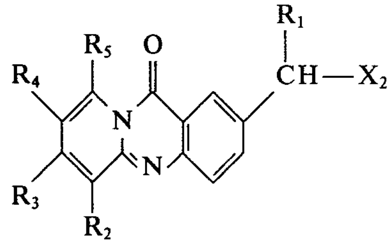

(I a)

worin

$R_1$ bis $R_5$ die in Anspruch 1 genannte Bedeutung besitzen und
$X_2$ eine Alkoxycarbonylgruppe mit 1 bis 6 Kohlenstoffatomen oder eine Carboxylgruppe und deren Salze mit physiologisch verträglichen Basen bedeutet, dadurch gekennzeichnet,

a) daß man ein Nitril der allgemeinen Formel I b

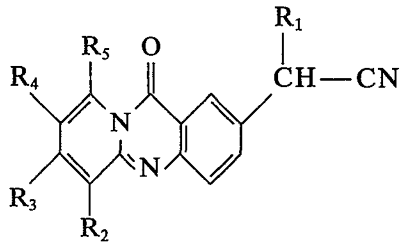

(Ib)

worin

$R_1$ bis $R_5$ die obengenannte Bedeutung besitzen, hydrolysiert, oder

b) daß man eine Carbonsäure der allgemeinen Formel IV

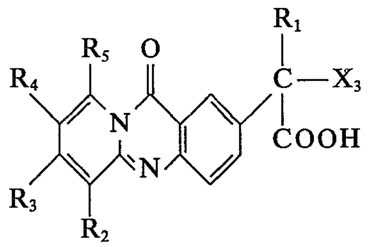

(IV)

worin

$R_1$ bis $R_5$ die obengenannte Bedeutung besitzen und
$X_3$ eine Cyanogruppe, Carboxylgruppe oder Alkoxycarbonylgruppe darstellt, decarboxyliert und gegebenenfalls hydrolysiert, oder

c) daß man eine Verbindung der allgemeinen Formel V

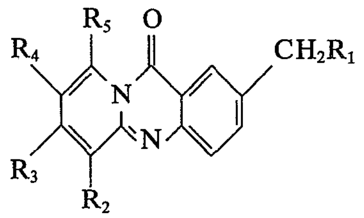

(V)

worin

$R_1$ bis $R_5$ die obengenannte Bedeutung besitzen,

in Gegenwart von Alkalimetallhydriden, Alkalimetallamiden oder Alkalimetallalkoholaten mit Kohlendioxid oder einem Chlorkohlensäurealkylester umsetzt, sowie gegebenenfalls die racemische Carbonsäure in ihre optischen Antipoden aufspaltet und/oder die Carbonsäure oder reaktionsfähige Derivate derselben in ihre Salze, Ester und Amide überführt und gewünschtenfalls die so erhaltenen Pyrido[2,1-b]chinazolinon-Derivate mit physiologisch unbedenklichen Säuren umsetzt.

**Claims**

1. Pyrido[2,1-b]quinazolinone derivates of the general formula I

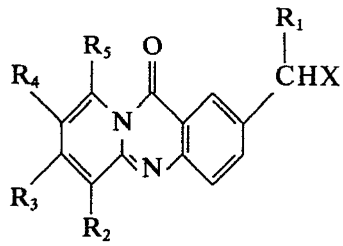

(I)

in which

X represents a cyano group, a carbamoyl group, an alkoxycarbonyl group having from 1 to 6 carbon atome in the alkyl radical or a carboxyl group, and the salts thereof with physiologically tolerable bases,

$R_1$ represents a hydrogen atom or a methyl group and

$R_2$ to $R_5$ represent hydrogen atoms, halogen atoms, alkyl groups containing from 1 to 4 carbon atoms, methylthio groups, methylsulphinyl groups or methylsilphonyl groups, with the proviso that 2 or 3 of the groups $R_2$ to $R_5$ represent hydrogen atoms,

and the salts thereof with physiologically tolerable acids or bases.

2. Pyrido[2,1-b]quinazolinone derivates of the general formula I according to claim 1 in which 1 or 2 of the groups $R_2$ to $R_5$ represent fluorine atoms, chlorine atoms, bromine atoms and/or methyl groups.

3. Pyrido[2,1-b]quinazolinone derivates of the general formula I according to claims 1 and 2, in which X represents a carbonxyl group, or represents the alkali metal salt, alkaline earth metal salt, copper(II) salt or methylglucamine salt thereof.

4. 2-(8-bromo-11-oxo-11H-pyrido[2,1-b]quinazolin-2-yl)propionic acid methyl ester.

5. 2-(8-chloro-11-oxo-11H-pyrido[2,1-b]quinazolin-2-yl)propionic acid methyl ester.

6. 2-(8-fluoro-11-oxo-11H-pyrido[2,1-b]quinazolin-2-yl)propionic acid methyl ester.

7. 2-(8-mesylamino-11-oxo-11H-pyrido[2,1-b]quinazolin-2-yl)propionic acid methyl ester.

8. 8-bromo-11-oxo-11H-pyrido[2,1-b]quinazoline-2-acetonitrile.

9. 8-bromo-11-oxo-11H-pyrido[2,1-b]quinazoline-2-acetic acid.

10. 2-(8-bromo-11-oxo-11H-pyrido[2,1-b]quinazolin-2-yl)propionic acid.

11. 2-(8-chloro-11-oxo-11H-pyrido[2,1-b]quinazolin-2-yl)propionic acid.

12. 2-(6-chloro-11-oxo-11H-pyrido[2,1-b]quinazolin-2-yl)propionic acid methyl ester.

13. 2-(6-chloro-11-oxo-11H-pyrido[2,1-b]quinazolin-2-yl)propionic acid.

14. 2-(8-fluoro-11-oxo-11H-pyrido[2,1-b]quinazolin-2-yl)propionic acid.

15. 2-(6,8-dichloro-11-oxo-11H-pyrido[2,1-b]quinazolin-2-yl)propionic acid methyl ester.

16. 2-(6,8-dichloro-11-oxo-11H-pyrido[2,1-b]quinazolin-2-yl)propionic acid.

17. 2-(7-methyl-11-oxo-11H-pyrido[2,1-b]quinazolin-2-yl)propionic acid methyl ester.

18. 2-(7-methyl-11-oxo-11H-pyrido[2,1-b]quinazolin-2-yl)propionic acid.

19. 2-(9-methyl-11-oxo-11H-pyrido[2,1-b]quinazolin-2-yl)propionic acid methyl ester.

20. 2-(9-methyl-11-oxo-11H-pyrido[2,1-b]quinazolin-2-yl)propionic acid.

21. 2-(8-methyl-11-oxo-11H-pyrido[2,1-b]quinazolin-2-yl)propionic acid methyl ester.

22. 2-(8-methyl-11-oxo-11H-pyrido[2,1-b]quinazolin-2-yl)propionic acid.

23. 2-(8-bromo-7-methyl-11-oxo-11H-pyrido[2,1-b]quinazolin-2-yl)propionic acid methyl ester.

24. 2-(8-bromo-7-methyl-11-oxo-11H-pyrido[2,1-b]quinazolin-2-y)propionic acid.

25. 2-(8-chloro-6-methyl-11-oxo-11H-pyrido[2,1-b]quinazolin-2-yl)propionic acid.

26. 2-(8-methylthio-11-oxo-11H-pyrido[2,1-b]quinazolin-2-yl)propionic acid methyl ester.

27. 2-(8-methylthio-11-oxo-11H-pyrido[2,1-b]quinazolin-2-yl)propionic acid.

28. 2-(8-methylsulphinyl-11-oxo-11H-pyrido[2,1-b]quinazolin-2-yl)propionic acid.

29. 2-(8-methylsulphinyl-11-oxo-11H-pyrido[2,1-b]quinazolin-2-yl)propionic acid methyl ester.

30. Pharmaceutical preparations characterised by a content of one or two pyrido[2,1-b]quinazolinone derivates according to claims 1 to.

31. Process for the manufacture of pyrido[2,1-b]quinazolinone derivates according to claim 1, characterised in that a pyridine derivate of the general formula II is condensed with a phenyl derivative of the general formula III

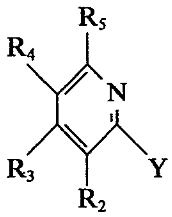

(II)

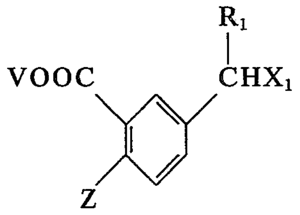

(III)

in which

$R_1$ to $R_5$ have the meanings given in claim 1 and
$X_1$ represents a cyano group, a carboxyl group or an alkoxycarbonyl group,
V represents a hydrogen atom or an alkyl group,
Y represents an amino group and Z represents a halogen atom or
Y represents a halogen atom, an esterified hydroxy group or an etherified hydroxy group and
Z represents an amino group,

the resulting cyano compound or alkoxycarbonyl compound is optionally hydrolysed or convertes into the carbamoyl compound, and the racemic carboxylic acid is optionally split into its optical antipodes and/or the carboxylic acids or reactive derivatives thereof are converted into their salts, esters or amides and, if desired, the resulting pyrido[2,1-b]quinazolinone derivatives are reacted with physiologically tolerable acids and bases.

32. Process for the manufacture of pyrido[2,1-b]quinazolinone derivatives of the general formula I a

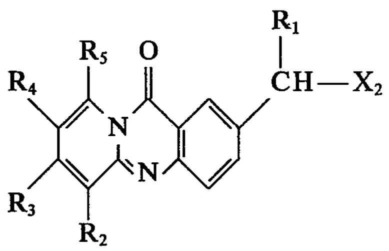

(Ia)

in which

$R_1$ to $R_5$ have the meanings given in claim 1 and
$X_2$ represents an alkoxycarbonyl group having from 1 to 6 carbon atoms or a carboxyl group and the salts thereof with physiologically tolerable bases,
characterised in that

a) a nitrile of the general formula I b

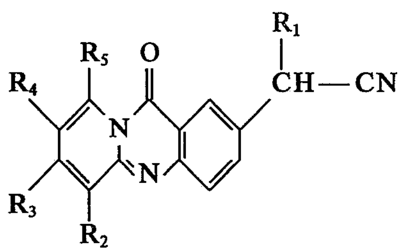

(Ib)

in which

$R_1$ to $R_5$ have the meaning given above, is hydrolysed or

b) a carboxylic acid of the general formula IV

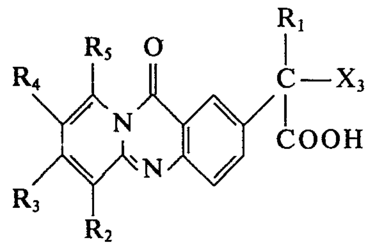

(IV)

in which

$R_1$ to $R_5$ have the meaning given above and

$X_3$ represents a cyano group, a carboxyl group or an alkoxycarbonyl group, is decarboxylated and optionally hydrolysed, or

c) a compound of the general formula V

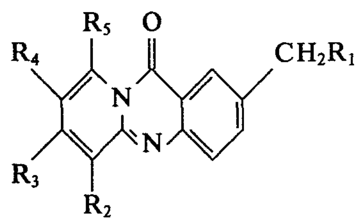

(V)

in which

$R_1$ to $R_5$ have the meaning given above,

is reacted in the presence of alkali metal hydrides, alkali metal amides or alkali metal alcoholates with carbon dioxide or a chlorocarbonic acid alkyl ester, and the racemic carboxylic acid is optionally split into its optical antipodes and/or the carboxylic acid or reacitve derivatives thereof are converted into their salts, esters and amides and, if desired, the resulting pyrido[2,1-b]quinazolinone derivatives are reacted with physiologically tolerable acids.

**Revendications**

1. Dérivés de la pyrido[2,1-b]quinazolinone répondant à la formule I

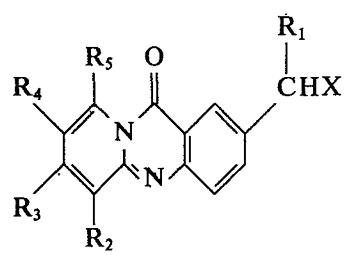

(I)

dans laquelle

X représente un radical cyano, un radical carbamoyle, un radical alcoxycarbonyle contenant de 1 à 6 atomes de carbone dans la partie alkylique ou un radical carboxy éventuellement sous la forme d'un sel ave une base acceptable du point de vue physiologique,

$R_1$ représente un atome d'hydrogène ou un radical méthyle et

$R_2$ à $R_5$ représentent des atomes d'hydrogène, des atomes d'halogènes, des radicaux alkyles contenant de 1 à 4 atomes de carbone, ou des radicaux méthylthio, méthylsulfinyles ou méthylsulfonyles, avec la restriction que 2 ou 3 des symboles $R_2$ à $R_5$ représentent des atomes d'hydrogène,

ainsi que les sels qu'ils forment avec des acides ou des bases inoffensives du point de vue physiologique.

2. Dérivés de la pyrido[2,1-b]quinazolinone de formule générale I selon la revendication 1, dans

lesquels 1 ou 2 des symboles $R_2$ à $R_5$ représentent des atomes de fluor, des atomes de chlore, des atomes de brome et/ou des radicaux méthyles.

3. Dérivés de la pyrido[1,1-b]quinazolinone de formule générale I selon l'une des revendications 1 et 2, dans lesquels X représente un radical carboxy éventuellement sous la forme d'un sel formé avec un métal alcalin, un métal alcalinoterreux, leur sel de cuivre(II) ou leur sel de méthyl-glucamine.

4. (bromo-8-oxo-11-11H-pyrido[2,1-b]quinazolinyl-2)-2-propionate de méthyle.

5. (chloro-8-oxo-11-11H-pyrido[2,1-b]quinazolinyl-2)-2 propionate de méthyle.

6. (fluoro-8-oxo-11-11H-pyrido[2,1-b]quinazolinyl-2)-2 propionate de méthyle.

7. (méthylsulfonylamino-8-oxo-11-11H-pyrido[2,1-b]quinazolinyl-2)-2 propionate de méthyle.

8. (bromo-8-oxo-11-11H-pyrido[2,1-b]quinazolinyl-2)-acétonitrile.

9. acide (bromo-8-oxo-11-11H-pyrido[2,1-b]quinazolinyl-2)-acétique.

10. acide (bromo-8-oxo-11-11H-pyrido[2,1-b]quinazolinyl-2)-propionique.

11. acide (chloro-8-oxo-11-11H-pyrido[2,1-b]quinazolinyl-2)-2 propionique.

12. (chloro-6-oxo-11-11H-pyrido[2,1-b]quinazolinyl-2)-2 propionate de méthyle.

13. acide (chloro-6-oxo-11-11H-pyrido[2,1-b]quinazolinyl-2)-2 propionique.

14. acide (fluoro-8-oxo-11-11H-pyrido[2,1-b]quinazolinyl-2)-2 propionique.

15. (dichloro-6,8-oxo-11-11H-pyrido[2,1-b]quinazolinyl-2)-2 propionate de méthyle.

16. acide (dichloro-6,8-oxo-11-11H-pyrido[2,1-b]quinazolinyl-2)-2 propionique.

17. (méthyl-7-oxo-11-11H-pyrido[2,1-b]quinazolinyl-2)-2 propionate de méthyle.

18. acide (méthyl-7-oxo-11-11H-pyrido[2,1-b]quinazolinyl-2)-2 propionique.

19. (méthyl-9-oxo-11-11H-pyrido[2,1-b]quinazolinyl-2)-2 propionate de méthyle.

20. acide (méthyl-9-oxo-11-11H-pyrido[2,1-b]quinazolinyl-2)-2 propionique.

21. (Méthyl-8-oxo-11-11H-pyrido[2,1-b]quinazolinyl-2)-2 propionate de méthyle.

22. Acide (méthyl-8-oxo-11-11H-pyrido[2,1-b]quinazolinyl-2)-2 propionique.

23. (Bromo-8-méthyl-7-oxo-11-11H-pyrido[2,1-b]quinazolinyl-2)-2 propionate de méthyle.

24. Acide (bromo-8-méthyl-7-oxo-11-11H-pyrido[2,1-b]quinazolinyl-2)-2 propionique.

25. Acide (chloro-8-méthyl-6-oxo-11-11H-pyrido[2,1-b]quinazolinyl-2)-2 propionique.

26. (Méthylthio-8-oxo-11-11H-pyrido[2,1-b]quinazolinyl-2)-2 propionate de méthyle.

27. Acide (méthylthio-8-oxo-11-11H-pyrido[2,1-b]quinazolinyl-2)-2 propionique.

28. Acide (méthylsulfinyl-8-oxo-11-11H-pyrido[2,1-b]quinazolinyl-2)-2 propionique.

29. (Méthylsulfinyl-8-oxo-11-11H-pyrido[2,1-b]quinazolinyl-2)-2 propionate de méthyle.

30. Compositions pharmaceutiques caractérisées en ce qu'elles contiennent un ou deux dérivés de la pyrido[2,1-b]quinazolinone selon l'une quelconque des revendications 1 à 30.

31. Procédé de préparation de dérivés de la pyrido[2,1-b]quinazolinone selon la revendication 1, procédé caractérisé en ce qu'on condense un dérivé de la pyridine de formule générale II avec un composé phénylique de formule générale III

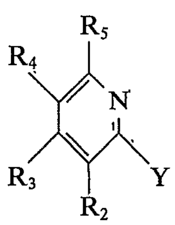

(II)

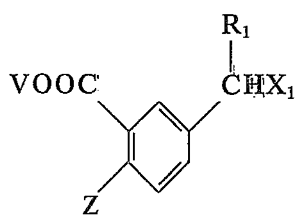

(III)

dans lesquelles

$R_1$ à $R_5$ ont significations données à la revendication 1,
$X_1$ représente un radical cyano, un radical carboxy ou un radical alcoxycarbonyle,
V représente un atome d'hydrogène ou un radical alkyle,
Y représente un groupe amino et Z un atome d'halogène, ou
Y représente un atome d'halogène ou un groupe hydroxy estérifié ou éthérifié et
Z un groupe amino,

éventuellement on saponifie le composé cyano ou alcoxycarbonylique ainsi formé ou on le convertit en le composé carbamoylique, le cas échéant on dédouble l'acide carboxylique racémique en ses antipodes optiques et/ou on transforme les acides carboxyliques ou des dérivés réactifs de ceux-ci en leurs sels, esters ou amides et, si on le désire, on fait réagir les dérivés de la pyrido[2,1-b]quinazolinone ainsi obtenus avec des acides ou des bases physiologiquement inoffensives.

32. Procédé de préparation de dérivés de la pyrido[2,1-b]quinazolinone répondant à la formule générale Ia

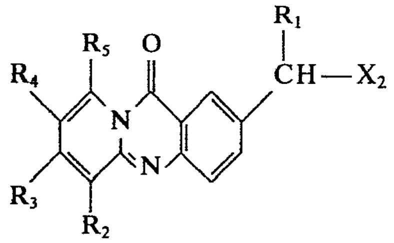

(Ia)

dans laquelle

$R_1$ à $R_5$ ont les significations données à la revendication 1 et

$X_2$ représente un radical alcoxycarbonyle contenant de 1 à 6 atomes de carbone ou un groupe carboxy éventuellement sous la forme d'un sel avec une base acceptable du point de vue physiologique,

procédé caractérisé en ce que:

a) on hydrolyse un nitrile répondant à la formule générale Ib

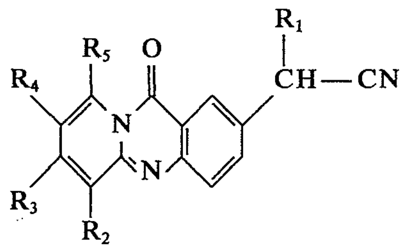

(Ib)

dans laquelle

$R_1$ à $R_5$ ont les significations précédemment données, ou

b) on décarboxyle et éventuellement hydrolyse un acide carboxylique répondant à la formule IV

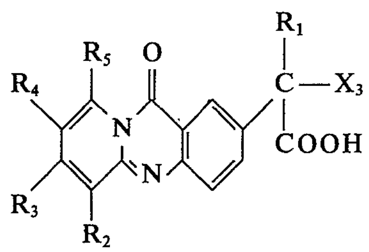

(IV)

dans laquelle

$R_1$ à $R_5$ ont les significations précédemment données et

$X_3$ représente un radical cyano, un radical carboxy ou un radical alcoxycarbonyle, ou

c) on fait réagir un composé répondant à la formule générale V

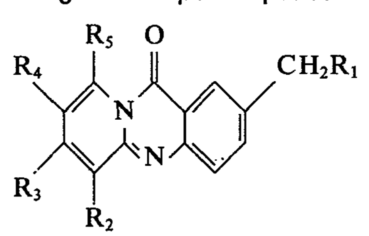

(V)

dans laquelle

$R_1$ à $R_5$ ont les significations précédemment données,

en présence d'hydrures de métaux alcalins, d'amidures de métaux alcalins ou d'alcoolates de métaux alcalins, avec le dioxyde de carbone ou un chloroformiate d'alkyle, éventuellement on dédouble l'acide carboxylique racémique en ses antipodes optiques et/ou on convertit l'acide carboxylique ou un dérivé réactif de celui-ci en ses sels, esters ou amides et éventuellement on fait réagir les dérivés de la pyrido[2,1-b]quinazolinone ainsi obtenus avec des acides inoffensifs du point de vue physiologique.